# EUROPEAN PATENT SPECIFICATION

(11) **EP 1 642 601 B1**
(45) Date of publication and mention of the grant of the patent: **09.07.2014**
(21) Application number: 05255977.0
(22) Date of filing: 26.09.2005
(51) Int. Cl.: A61L 27/50, A61L 27/58, A61L 31/14, A61B 17/04

(54) **Suture anchor and void filler combination**
Kombination von Fadenanker und Knochenfüller
Combinaison d'un ancrage de suture et d'un matériau de comblement osseux

(30) Priority: 27.09.2004 US 951012
(43) Date of publication of application: 05.04.2006
(73) Proprietor: ETHICON, INC., Somerville, NJ 08876 (US)
(72) Inventor: Story, Brooks J., Franklin, MA 02038 (US); Schachter, Deborah, Edison, NJ 08817 (US); McAlister, Gary, Franklin, MA 02038 (US); Skula, Richard E., New Jersey 07470 (US)
(74) Representative: Tunstall, Christopher Stephen

(56) References cited:
- EP-A- 0 727 230
- WO-A-01/43625
- WO-A-02/00119
- WO-A2-03/059409
- US-A- 5 681 873
- US-A1- 2002 187 104

## Description

### Technical Field

The field of art to which this invention relates is sports medicine, more particularly, suture anchors for approximating soft tissue to bone, bone void fillers and surgical procedures using suture anchors and bone void fillers.

### Background of the Invention

Surgical suture anchors for the approximation of soft tissue to the surface of a bone are well known in the art. Suture anchors are typically used in sports medicine surgical procedures to repair soft tissue in damaged joints, for example, the rotator cuff in the shoulder. Suture anchors may have a variety of known configurations including threaded screws, wedges, cylindrical members with Nitnol wire tangs, rivets, plugs, etc. The suture anchors may be made of conventional nonabsorable biomaterials such as surgical stainless steel, titanium, Nitinol, etc. The anchors may also be made from conventional bioabsorbable or bioresorbable (i.e., biodegradable) materials such as polymers and copolymers of lactic acid, dioxanone, caprolactone, gylcolide, glycolic acid, etc. Suture anchors, methods of using suture anchors, and materials for constructing suture anchors are disclosed in the following United States patents:
U.S. Patent Nos. 4,632,100, 4,999,074, 5,814,051, 5,709,708, 5,782,864, 6,270,518, 5,540,718, 6,264,674, 6,270,518, 6,306,158, 5,961,538, 5,782,863, 5,683,418, 5,554,171, 5,078,730, 4,632,100, 5,217,486, 5,011,473, 4,898,156, 4,899,743, 4,946,468,4,968,315, 5,002,550, 5,046,513, 5,192,303, 5,207,679, 5,358,511.

Suture anchors may be implanted using conventional open or arthroscopic surgical procedures. The orthopedic surgeon typically prefers to use minimally invasive, arthroscopic techniques because of the benefits to the patient. Such benefits may include reduced pain, minimal incision size, reduced incidence of infection, the use of local versus general anesthesia, reduced procedure time, reduced scarring, and improved recovery time. In a typical, conventional surgical procedure to repair a soft tissue injury wherein a suture anchor is to be implanted, the surgeon drills a bore hole in a bone adjacent to a site where the soft tissue is to be approximated to the surface of the bone to effect the repair. This is done using conventional surgical drills and techniques. The bore hole preferably is a "blind" hole having a bottom. A surgeon typically implants a suture anchor into a bore hole by mounting the anchor to the distal end of an elongated insertion member such as a rod, and then inserting the anchor into the bore hole. Once the anchor is secured in the bore hole, in cancellous bone beneath the outer cortex, the insertion member is detached from the anchor, the anchor is set in a fixed position within the bore hole, and the anchor installation is then complete. The affixation of soft tissue to bone is accomplished by using a surgical suture in combination with the suture anchor and preferably mounted to the anchor, wherein the surgical suture has at least one surgical needle attached, preferably a surgical needle is attached to each end of the suture. It is also possible and often desirable to combine and/or mount more than one suture with or to the suture anchor. The suture and needle are mounted to the suture anchor prior to insertion of the suture anchor into the bore hole. The surgeon uses the needle(s) and suture(s) to penetrate the soft tissue and approximate and secure the soft tissue to the surface of the bone, thereby completing the repair. More than one suture anchor may be necessary to provide for a sufficiently adequate repair. One of the advantages of the use of suture anchors to affix soft tissue to bone is the elimination of the need for bone tunnels. Bone tunnels are open-ended tunnels drilled through a bone so that a surgical suture can be passed through the tunnel for use in approximating soft tissue to the bone surface. The use of bone tunnels is known to have several disadvantages including weakening the bone structure, providing a site for infections to occur, increasing the duration of the surgical procedure and the so called "cheesewire effect" in which the suture is pulled through the bone in which the tunnel is created, resulting in a failure of the re-attachment procedure. The use of suture anchors eliminates many of these disadvantages and generally provides superior soft tissue fixation.

When a suture anchor is mounted in a bone bore hole, the volume of the anchor is typically substantially less than the overall volume of the bone bore hole, resulting in a void volume in the bone bore hole that is equal to the volume of the bore hole minus the volume of the suture anchor. Over time, the natural healing response of the patient's body will often cause the void volume of the bore hole to be filled in with new bone tissue resulting in the anchor being substantially surrounded by the new bone tissue. In the case of bioabsorbable or resorbable anchor bodies, the new bone tissue will also replace the anchor volume as it is absorbed or resorbed. The ingrowth of new bone tissue is desirable for a number of reasons. It is generally believed that it is not desirable to leave a bone void in a bone after a surgical procedure. Thus, there are several deficiencies that may be associated with the presence of void volume in a bone bore hole. The void volume may compromise the integrity of the bone, resulting in structural weakening, thereby making the bone possibly susceptible to fracture until the void volume becomes ingrown with native bone. The void volume may also provide an opportunity for the incubation and proliferation of any infective agents that are introduced during the surgical procedure, and is also susceptible to infectious agents carried by body fluids into the void volume. In addition, it is possible that the bone void volume may not heal completely.

A common side effect of any surgery is ecchymosis in the surrounding tissue which results from bleeding of the traumatized tissues. Finally, the surgical trauma to the bone and the overlying periosteum is known to be a significant source of postoperative pain and inflammation. In addition to the extreme discomfort, post-operative pain and inflammation severely limit the patient's range of motion, thereby delaying their return to function. It is known that the healing process is facilitated by an early return to limited motion thus, alleviation of pain and swelling will facilitate the post-operative healing process.

Accordingly, there is a need in this art for novel suture anchor combinations and surgical procedures that provide for immediate filling of the void volume in a bone bore hole, and that promote a rapid ingrowth of new native bone into the bore hole, and which could prevent or alleviate pain, inflammation and potential infection potentially resulting from a surgical procedure.

WO02/00119 discloses a method of securing a fixation device within an opening in a tissue, including delivering a material in a flowable state to said opening, and changing the state of the material so that the material forms an interference fit that secures the fixation device in the opening. WO03/059409 discloses a biodegradable implant material, the material comprising anhydrous calcium sulphate in the form of soluble or insoluble anhydrite.

### Summary of the Invention

A bone void filler composition is disclosed. The composition is for filling at least a portion of a bone void volume containing a suture anchor which has an anchor body. The anchor body has a volume. The composition comprises a biodegradable material, and it is for delivery to the bone void volume as a solid. The biodegradable material comprises a member selected from the group consisting of hydroxyethyl cellulose and hyaluronic acid. The composition additionally comprises an effective amount of a therapeutic agent.

The bone void filler composition optionally contains osteoinductive and/or osteoconductive materials.

These and other aspects and advantages of the present invention will become more apparent by the following description and accompanying drawings.

### Brief Description of the Drawings

FIG. 1 illustrates a cross-section of a bone prior to drilling a bone bore hole.
FIG. 2 illustrates a drill as it drills a bone bore hole in the bone.
FIG. 3 illustrates the bore hole in the bone, after the drill has been removed.
FIG. 4 illustrates the bore hole of FIG. 3 after a conventional suture anchor has been inserted.
FIG 5. illustrates the anchor and bore hole of FIG. 4 after a bone void filler composition has been inserted, thereby substantially filling in the bore hole void.
FIG. 6 illustrates the bore hole, anchor and bone void filler composition of FIG. 5 after the suture mounted to the anchor has been used by the surgeon to affix soft tissue to the surface of the bone, and complete the surgical repair.
FIG. 7 illustrates the bone of FIG. 7 after new bone has ingrown and replaced the void filler about the suture anchor in the bone bore hole.

### Disclosure of Preferred Embodiment

The suture anchors that can be used in connection with the bone void filler composition of the present invention include any conventionally available and known suture anchors, and equivalents thereof. Such suture anchors include but are not limited to anchors having wedge-shaped bodies, screw threaded anchors, anchors having cylindrical bodies with resilient bone engaging members extending from the bodies, rivet-type anchors, plug anchors, force-fit anchors, compressible anchors, anchors with bone engaging members or projections, etc. Suture anchors may be made from a variety of absorbable and nonabsorbable biomaterials including, but not limited to, surgical stainless steel, titanium, Nitinol, polymers such as aliphatic polyesters, polyorthoesters, polyanhydrides, polycarbonates, polyurethanes, polyamides and polyalkylene oxides. The aliphatic polyesters are typically synthesized in a ring opening polymerization. Suitable monomers include but are not limited to lactic acid, lactide (including L-, D-, meso and D,L mixtures), glycolic acid, glycolide, e-caprolactone p-dioxanone (1,4-dioxan 2 one), trimethylene carbonate (1,3-dioxan-2-one), , delta-valerolactone, beta-butyrolactone, epsilon-decalactone, 2,5-diketomorpholine, pivalolactone, α,alpha-diethylpropiolactone, ethylene carbonate, ethylene oxalate, 3-methyl-1, 4-dioxane-2,5-dione, 3,3-diethyl-1,4-dioxan-2,5-dione, gamma-butyrolactone, 1,4-dioxepan-2-one, 1,5-dioxepan-2-one, 6,6-dimethyl-dioxepan-2-one, 6,8-dioxabicycloctane-7-one,combinations thereof and the like. Suture anchors and materials for constructing suture anchors are disclosed in the following United States patents:
U.S. Patent Nos. 4,632,100, 4,999,074, 5,814,051, 5,709,708, 5,782,864, 6,270,518, 5,540,718, 6,264,674, 6,270,518, 6,306,158, 5,961,538, 5,782,863, 5,683,418, 5,554,171, 5,078,730, 4,632,100, 5,217,486, 5,011,473, 4,898,156, 4,899,743, 4,946,468, 4,968,315, 5,002,550, 5,046,513, 5,192,303, 5,207,679, 5,358,511.

The term biodegradable as used herein is defined to include both bioabsorbable and bioresorbable materials. By biodegradable, it is meant that the materials are degraded or broken down (chemically or physically) under physiological conditions in the body such that the degradation products are excretable or absorbable by the body.

The bone void filler compositions of the present invention are made from biodegradable materials known in this art.

It is particularly preferred to use a void filler consisting of hydroxyethyl cellulose (HEC). Typically the bone void filler compositions of the present invention will contain about 5 to about 99 weight percent of biodegradable material, more typically about 15 to about 75 weight percent, and preferably about 15 to about 55 weight percent. When the bone void filler compositions also contain a therapeutic agent, then the bone void filler compositions will contain a sufficient amount of biodegradable polymer to effectively allow release of an effective amount of therapeutic agent in the region surrounding the bone void.

The bone void filler compositions of the present invention are used in a solid state such as powders, granules, tablets, capsules, extruded rods, molded or machined shapes and structures, etc. Powder or granules may be tamped in place, powder or granules may be compressed into a tablet and placed into the void space, extruded plugs may be placed into the void space, a molded bolus or structure may be placed into the void space, etc.

The bone void filler compositions of the present invention may optionally contain a variety of osteoinductive materials to accelerate of ingrowth of bone.. Examples of osteoinductive materials suitable for use with the present invention include cell attachment mediators, such as peptide-containing variations of the "RGD" integrin binding sequence known to affect cellular attachment, biologically active ligands, and substances that enhance or exclude particular varieties of cellular or tissue ingrowth. Examples of such substances include integrin binding sequence, ligands, bone morphogenic proteins, epidermal growth factor, IGF-I, IGF-II, TGF-β I-III, growth differentiation factor, parathyroid hormone, vascular endothelial growth factor, hyaluronic acid, glycoprotein, lipoprotein, bFGF, TGF-β superfamily factors, BMP-2, BMP-4, BMP-6, BMP-12, sonic hedgehog, GDF5, rhGDF5, GDF6, GDF8, PDGF, small molecules that affect the upregulation of specific growth factors, tenascin-C, fibronectin, thromboelastin, thrombin-derived peptides, heparin-binding domains, and the like. Furthermore, the bone replacement material may comprise mineralized collagen particles mixed with a biologically derived substance selected from the group consisting of demineralized bone matrix (DBM), platelet rich plasma, bone marrow aspirate and bone fragments, all of which may be from autogenic, allogenic, or xenogenic sources. A therapeutically effective amount of the osteoinductive materials is incorporated into the bone void filler compositions. The amount of osteoinductive material in the void filler compositions of the present invention will be sufficient to effectively provide for accelerated bone in-growth into a void volume. The amount of osteoinductive material will typically be about 0.01 weight percent to about 1 weight percent.

The bone void filler compositions may contain a sufficiently effective amount of an osteoconductive material to provide for accelerated bone ingrowth into the void volume. The and osteoconductive materials include, but are not limited to, alpha-tricalcium phosphate (alpha-TCP), beta-tricalcium phosphate (beta-TCP), calcium carbonate, barium carbonate, calcium sulfate, barium sulfate, hydroxyapatite, and mixtures thereof. In certain embodiments the inorganic filler comprises a polymorph of calcium phosphate, equivalents thereof, combinations thereof and the like. A particularly preferred material is beta-TCP. The amount of the osteoconductive material in the void filler compositions will typically range from about 5 to about 50 weight percent, more typically about 10 to about 40 weight percent, and preferably about 20 to about 30 weight percent. The amount of osteoconductive material in the void fillers of the present invention will be sufficient to effectively conduct bone growth into the void space.

The bone void filler compositions of the present invention may also include a conventional high molecular weight hydrophilic polymer that can regulate the release rate of a pharmaceutical agent in the void filler composition. Such hydrophilic polymers include polysaccharides, chitins and derivatives, hyaluronic acids and derivatives, hydroxyethylcellulose, hydroxypropylmethylcellulose, hydroxymethylcellulose, hydroxypropylcellulose, carboxymethylcellulose, alginates, polyvinylpyrrolidone, polyethylene oxide, polyethylene glycol, polyacrylic acid and derivatives, gums (i.e. guar, carob bean), polymers derived from starch. These polymers can be combined with other components of the formulation either by direct mixing of powders, melt processing, or wet granulation. The solid mixture can be delivered to the void space where it is exposed to physiological fluid and can hydrate into a hydrogel. The amount or molecular weight of the hydrophilic polymer can be used to determine the rigidity of the resulting hydrogel as well as the release rate of an active agent contained within it. Increasing molecular weight results in a decrease in the rate of release.

To extend the timed release of the drug to beyond the length of time necessary for diffusion from or erosion of hydrophilic polymer it is possible to disperse within the hydrophilic polymer a hydrophobic absorbable polymer that also contains the active agent. This can be achieved by melt-processing the hydrophobic polymer, active agent, and the hydrophilic polymer together in an extruder and placing the plug cut from the extrudate directly into the void space. Here, again, the hydration of the hydrophilic polymer into a gel is fast and dispersed within this gel matrix are domains of the hydrophobic polymer containing more active agent. The active agent is partly in the hydrophilic matrix from which a higher concentration of active can be released sooner and partly in the hydrophobic polymer matrix from which it is released slowly for a longer time period. In this case release rate of the active can be controlled by molecular weight of the hydrophilic polymer as well as the composition of the matrix (ratio of hydrophilic to hydrophobic). A sufficient amount of the hydrophilic polymer will be included in the bone void filler compositions to effectively provide for regulation of the rate of release of a drug or pharmaceutical agent incorporated into the void filler. The amount of hydrophilic polymer will typically be about 10 to about 70 weight percent, more typically about 15 to about 60 weight percent, and preferably about 15 to about 55 weight percent.

The void fillers of the present invention include one or more therapeutic agents. The therapeutic agents of the bone void filler compositions of the present invention include pain medications such as nonsteroidal anti-inflammatory drugs (NSAIDS), opioid analgesics (oxycodone, morphine, fentanyl, hydrocodone, naproxyphene, codeine, etc.), opioid/nonopioid combination analgesics (e.g. acetaminophen with codeine), acetaminophen; local anesthetics (benzocaine, lidocaine, procaine, bupivacaine, ropivacaine, mepivacaine, chloroprocaine, tetracaine, cocaine, etidocaine, prilocaine, procaine), alpha - 2 agonists (clonidine, xylazine, medetomidine, dexmedetomidine); VR1 antagonists; anti-infectives, such as antibiotics and antiviral agents; analgesics and analgesic combinations; anti-inflammatory agents; steroids, including corticosteroids; and, naturally derived or genetically engineered proteins, polysaccharides, glycoproteins, or lipoproteins. If desired multiple drugs may be included having the same or different indications.

The void fillers of the present invention can be sterilized by conventional processes and methods known in the art for sterilizing biodegradable polymers.

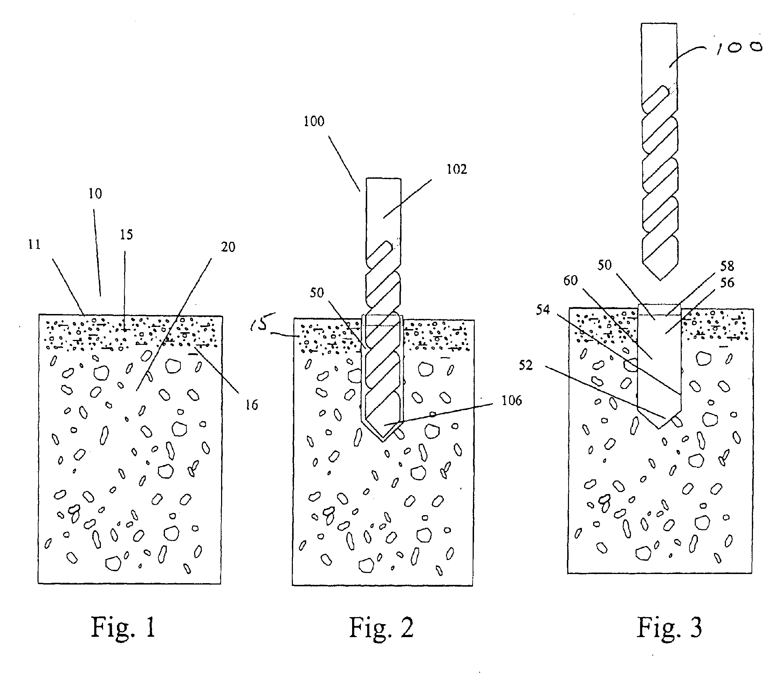

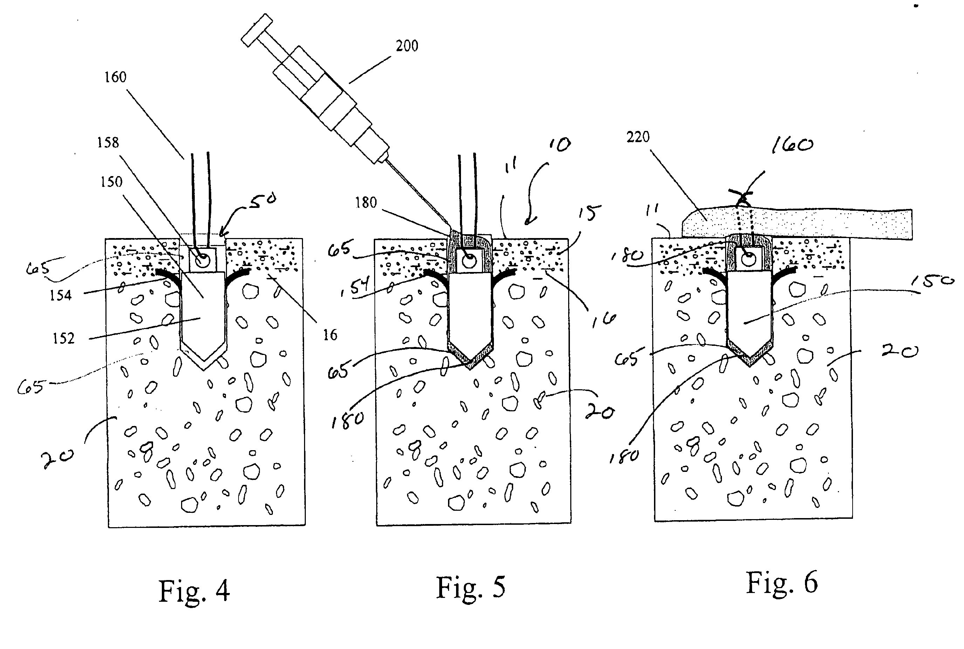

Referring now to FIGS. 1-7, the use of the present invention is illustrated. A cross-section of a typical bone section of a bone 10 is seen prior to drilling a bore hole in the bone 10. Bone 10 is seen to have surface 11, upper cortex layer 15 and interior cancellous layer 20. A conventional surgical bone drill 100 is seen in FIG.2 as it drills into the bone 10 to drill out the bone bore hole 50. Drill 100 is seen to have rod member 102 and distal cutting end 106. Bore hole 50 is seen in FIG. 3 after the drill cutting end 106 has been removed. Bore hole 50 is seen to have blind or closed distal bottom 52, side walls 54 and open proximal end 56 having opening 58 extending though top surface 11. The bore hole 50 is also seen to have bore hole volume 60. Referring to FIG. 4, conventional suture anchor 150 is seen mounted in bore hole 50. Suture anchor 150 is seen to partially fill up bore hole volume 60, resulting in void volume 65 . Void volume 65 is the volume resulting from the displacement of the bore hole volume 60 by the volume of anchor 150. The anchor 150 is seen to have cylindrical anchor body 152, flexible arc members 154 made from a material such as Nitinol Ni-Ti alloy, and suture mounting opening 158. A conventional surgical suture 160 is mounted to suture mounting opening 158. A conventional surgical suture 160 is mounted to suture mounting opening 158. The arc members 154 anchors are seen to engage the bottom 16 of cortex layer 15. Suture 160 is seen to extend from the bore hole 50 out through opening 58.

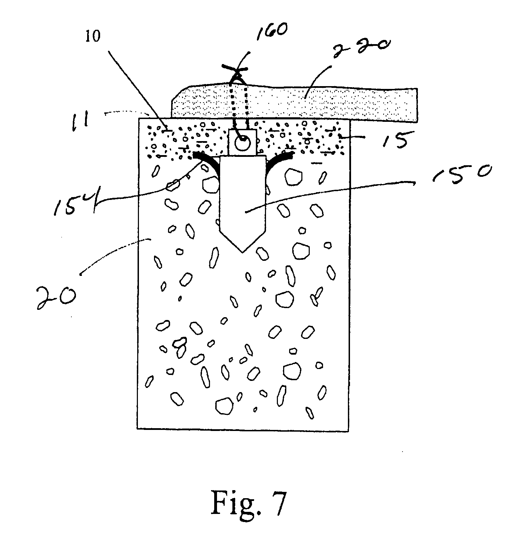

FIG. 5 illustrates the use of a bone void filler composition in liquid form, which is outside the scope of the invention. The syringe 200 is seen injecting the void filler 180 into the void volume 65 of bore hole 50 surrounding anchor 150. A sufficient amount of the bone void filler composition 180 is injected into the bore hole 50 to substantially fill in the remainder of the void volume 65, such that the filler 180 is in contact with anchor 150 and walls 54, completing the installation of the anchor and void filler composition combination. Preferably, the entire void volume 65 is filled. As seen in FIG. 6, soft tissue 220 is seen to be approximated against top surface 11 by suture 160, thereby completing the surgical soft tissue repair procedure. FIG. 7 illustrates the bone 10 after a sufficient healing period showing that the void filler 180 substantially replaced by ingrown native bone, with no remaining void volume 65.

As mentioned previously, the bone void filler composition 180 may also be applied by pouring a powder or granules into the void volume, tamping powders or granules into the void volume, compressing powders and/or granules into a tablet or other structure and placing it into the void volume, melt extruding plugs that can be placed into the void volume. If desired, the bone void filler compositions may be placed into the bore hole volume prior to inserting a suture anchor. For example, a tablet may be placed into the bore hole prior to inserting the anchor.

The following examples are illustrative of the principles and practice of the present invention, although not limited thereto.

### Example 1: Wet Granulation Method

A granulated void filler of the present invention was prepared in the following manner. Hydroxyethylcellulose (HEC) (Natrosol 250HHR; Hercules, Wilmington, DE) and tricalcium phosphate (TCP) (Tri-tab; Rhodia, Cranbury, NJ) were sieved respectively through a 45 mesh screen. A 1.8 gram quantity of the sieved TCP was dry-blended with 2.0 grams of lidocaine. A 1 milliliter aliquot of isopropanol was added to the dry-blended mixture dissolving the lidocaine (Sigma-Aldrich) and suspending the TCP particles. A 1.8 gram quantity of the sieved HEC was added, in small quantities, to this mixture, blending with a spatula after each addition. Mixing was continued until appearance was uniform. The granulated mixture was transferred to an aluminum pie pan and placed on a bench top to air dry for 3 hours. Further drying occurred overnight using a vacuum oven set at 40°C. After drying the mixture was in the form of white free-flowing granules. Granules can be used as is to pack a void or they could be compressed into a precisely shaped pellet to fit a void using a tablet press.

### Example 2: Melt Processing Method

A void filler useful in the practice of the present invention was prepared in the following manner. Hydroxyethylcellulose (HEC) (Natrosol 250HHR; Hercules, Wilmington, DE) and tricalcium phosphate (TCP) (Tri-tab; Rhodia, Cranbury, NJ) were sieved respectively through a 45 mesh screen. A 0.5 gram quantity of sieved TCP was dry-blended with 2.0 grams of lidocaine (Sigma-Aldrich), and 1 gram of the sieved HEC. 1.5 g of poly (caprolactone co-dioxanone) (PCL/PDS) (Ethicon; Somerville, NJ) in the mole ratio of 95/5 was weighed out. A twin screw extruder (DACA Instruments; Goleta, CA) was heated to 85°C and half of the PCL/PDS was fed into the extruder. Polymer was allowed to melt and mix for a few minutes. The dry blend was added slowly to the extruder. Then the remaining portion of the PCL/PDS was added. The mixture was processed in the extruder for 5 minutes under a nitrogen blanket. The load initially was 500 - 600 N but reduced to approximately 300 N during processing due to the melting of the lidocaine. The extrudate emerged as a thin translucent tacky rod. Upon cooling by contact with ambient atmosphere the extrudate turned an opaque off-white in color, most likely as a result of the crystallization of the PCL. The extruded rod was brittle when cool. The extrudate rod can be cut to fit a certain size void or chopped by an impeller into small particles resembling the granules in the example above. Alternatively, the powdered mixture can be mixed with the PCL/PDS and fabricated into a film using a compression molding process.

### Reference Example 3: Direct compression of powder method

A pelletized form of a void filler useful in the practice of the present invention was made in the following manner. Three grams of TCP (Tri-tab; Rhodia, Cranbury, NJ) and three grams of HEC (Natrosol 250HHR; Hercules, Wilmington, DE) were mixed in a 200 milliliter glass beaker with a spatula for five minutes. The powder mixture was milled in an IR ball mill (Spectra-Tech, Inc.) in 0.4 gram quantities for 30 seconds. A 0.2 gram amount of powder was placed in an IR pellet maker (Spectra-Tech, Inc.) and compressed at room temperature in a press (Fred S. Carver, Inc.; Summit, NJ) using a load of 1000 lbs for one minute. Pellet was removed from pellet maker.

### Reference Example 4: Direct compression of polyvinylpyrrolidone and TCP

A pelletized form of the void filler of the present invention was made in the following manner. Three grams of TCP (Tri-tab; Rhodia, Cranbury, NJ) and three grams of polyvinylpyrrolidone (K29/32; ISP, Wayne, NJ) were dry blended and compressed in the same manner as described in Example 3.

### Reference Example 5: Direct compression of hydroxypropylmethylcellulose (HPMC) and TCP

A pelletized form of a void filler of the present invention was manufactured in the following manner. Three grams of TCP ((Tri-tab; Rhodia, Cranbury, NJ) and three grams of HPMC (4000 cps, Sigma-Aldrich) were dry blended and compressed in the same manner as described in Example 3.

### Reference Example 6: Direct compression of HEC, TCP and sodium carboxymethylcellulose (CMC)

A pelletized form of a void filler of the present invention was prepared in the following manner. Three grams of TCP (Tri-tab; Rhodia, Cranbury, NJ), 1.5 grams of HEC (Natrosol 250HHR; Hercules, Wilmington, DE) and 1.5 grams of CMC (7HFPH; Hercules, Wilmington, DE) were dry blended and compressed in the same manner as described in Example 3.

### Reference Example 7: Melt processing of polymers and TCP

A solid rod of a void filler useful in the practice of the present invention was manufactured in the following manner. Hydroxyethylcellulose (HEC) (Natrosol 250HHR; Hercules, Wilmington, DE) and tricalcium phosphate (TCP) (Tri-tab; Rhodia, Cranbury, NJ) were sieved respectively through a 45 mesh screen. A 1.75 gram quantity of sieved TCP was dry-blended with 1.75 gram quantity of the sieved HEC. A 1.5 g amount of poly (caprolactone co-dioxanone) (PCL/PDS) (Ethicon; Somerville, NJ) in the mole ratio of 95/5 was weighed out. A twin screw extruder (DACA Instruments; Goleta, CA) was heated to 120°C and half of the PCL/PDS was fed into the extruder. Polymer was allowed to melt and mix for a few minutes. The dry blend was added slowly to the extruder followed by the remaining portion of the PCL/PDS. Mixing speed was 100 rpm and was conducted under a nitrogen blanket. Load was 5000 N. The extrudate emerged as a brittle rod that could be cut to size or chopped into small particles.

### Example 8

A patient is prepared for arthroscopic rotator cuff repair surgery in a conventional manner. Cannulas are placed into the patient's shoulder in a conventional manner for access to the operative site. A conventional arthroscope is inserted into the patient's shoulder in a conventional manner so that the surgical site can be viewed remotely by the surgeon. A sterile saline flow is established to insufflate the joint. The surgeon locates a site on the patient's proximal humerus to drill a bone bore hole on the greater tuberosity of the humerus. A conventional drill guide is inserted through one of the cannulas. A surgical drill is inserted through the drill guide, and the surgeon drills a bore hole into the bone. The drill is then removed from the bore hole. The bore hole has a top opening, a closed bottom and a void volume. The surgeon then inserts a conventional suture anchor into the bone bore hole using a conventional inserter. After the anchor is secured in the bone bore hole, the inserter is disengaged from the anchor and removed. The volume of the bone bore hole displaced by the volume of the anchor results in a void volume in the bone bore hole. The surgeon then inserts a sufficient amount of the bone void filler composition of Example 1 into the bone bore hole to effectively fill substantially all of the void volume. The surgeon then completes the repair by using surgical sutures mounted to the suture anchor to approximate the soft tissue (i.e., the rotator cuff tissue) to the surface of the bone. The cannulas are then withdrawn and the openings for the cannulas are approximated in a conventional manner by using bandages or sutures if necessary. The bore hole void is effectively filled in by the void filler composition. Elution of the desired therapeutic agent begins upon hydration of the void filler by body fluids. Following elution of the therapeutic agent and absorption of the biopolymer component, the remaining osteoconductive component promotes the in-growth of native bone to the bore hole.

The present invention has many advantages. The advantages include elimination of excess bone defect volume around implanted suture anchors, reduced likelihood of infection, alleviation of post-operative pain, and alleviation of post-operative swelling. The advantages also include reduced dependence on oral pain medications and/or external pain pumps, more rapid return to function, facilitation of physical therapy, more rapid mechanical reinforcement of anchor site due to enhanced bone ingrowth, controlled release of local medications, and, reduction of ecchymosis from bone defect bleeding.

## Claims

1. A bone void filler composition for use in a method for filling at least a portion of a bone void volume in a bore hole containing a suture anchor, wherein said anchor has an anchor body, said anchor body having a volume, and said void filler composition comprises a biodegradable material, **characterized in that** said method comprises delivering said composition to said bone void volume as a solid, said biodegradable material comprises a member selected from the group consisting of hydroxyethyl cellulose and hyaluronic acid, and said composition comprises an effective amount of a therapeutic agent.

2. A bone void filler composition as claimed in claim 1, wherein the bone void filler additionally comprises an osteoconductive component

3. A bone void filler composition as claimed in claim 2, wherein said osteoconductive component is selected from the group consisting of tricalcium phosphate, alpha-tricalcium phosphate, beta-tricalcium phosphate, calcium carbonate, barium carbonate, calcium sulfate, barium sulfate, hydroxyapatite, a polymorph of calcium phosphate, and combinations thereof.

4. A bone void filler composition as claimed in claim 3 wherein said osteoconductive component is beta-tricalcium phosphate.

5. A bone void filler composition as claimed in claim 1 wherein said therapeutic agent is selected from the group consisting of pain medication, antiinfectives, analgesics, anti-inflammatory agents, immunosupressives, steroids, including corticosteroids, glycoproteins, lipoproteins, and combinations thereof.

6. A bone void filler composition as claimed in claim 5 wherein said pain medication is selected from the group consisting of morphine, nonsteroidal anti-inflammatory drugs, oxycodone, morphine, fentanyl, hydrocodone, naproxyphene, codeine, acetaminophen with codeine, acetaminophen, benzocaine, lidocaine, procaine, bupivacaine, ropivacaine, mepivacaine, chloroprocaine, tetracaine, cocaine, etidocaine, prilocaine, procaine, clonidine, xylazine, medetomidine, dexmedetomidine, VR1 antagonists, and combinations thereof.

7. A bone void filler composition as claimed in claim 6 wherein said pain medication is bupivacaine.

8. A bone void filler composition as claimed in claim 1, wherein the bone void filler additionally comprises an osteoinductive component.

9. A bone void filler composition as claimed in claim 8, wherein said osteoinductive component is selected from the group consisting of cell attachment mediators, peptide-containing variations of the RGD integrin binding sequence known to affect cellular attachment, biologically active ligands, integrin binding sequence, ligands, bone morphogenic proteins, epidermal growth factor, IGF-I, IGF-II, TGF-β I-III, growth differentiation factor, parathyroid hormone, vascular endothelial growth factor, glycoprotein, lipoprotein, bFGF, TGF-β superfamily factors, BMP-2, BMP-4, BMP-6, BMP-12, BMP-14, sonic hedgehog, GDF6, GDF8, PDGF, tenascin-C, fibronectin, thromboelastin, thrombin-derived peptides, and heparin-binding domains.

10. A bone void filler composition as claimed in claims 1, 2 or 8, wherein said biodegradable material comprises 15 to 75 weight percent of the bone void filler composition.

## Patentansprüche

1. Knochenfüllerzusammensetzung für die Verwendung bei einem Verfahren zum Füllen wenigstens eines Teils eines Knochenhohlraumvolumens in einem Bohrloch, das einen Nahtanker enthält, wobei der Anker einen Ankerkörper aufweist, wobei der Ankerkörper ein Volumen aufweist und die Füllerzusammensetzung ein bioabbaubares Material umfasst, **dadurch gekennzeichnet, dass** das Verfahren das Abgeben der Zusammensetzung an das Knochenhohlraumvolumen als Feststoff umfasst, das bioabbaubare Material ein Element ausgewählt aus der Gruppe bestehend aus Hydroxyethylcellulose und Hyaluronsäure umfasst und die Zusammensetzung eine wirksame Menge eines therapeutischen Mittels umfasst.

2. Knochenfüllerzusammensetzung gemäß Anspruch 1, wobei der Knochenfüller zusätzlich eine osteokonduktive Komponente umfasst.

3. Knochenfüllerzusammensetzung gemäß Anspruch 2, wobei die osteokonduktive Komponente ausgewählt ist aus der Gruppe bestehend aus Tricalciumphosphat, alpha-Tricalciumphosphat, beta-Tricalciumphosphat, Calciumcarbonat, Bariumcarbonat, Calciumsulfat, Bariumsulfat, Hydroxyapatit, einem Polymorph von Calciumphosphat und Kombinationen davon.

4. Knochenfüllerzusammensetzung gemäß Anspruch 3, wobei die osteokonduktive Komponente beta-Tricalciumphosphat ist.

5. Knochenfüllerzusammensetzung gemäß Anspruch 1, wobei das therapeutische Mittel ausgewählt ist aus der Gruppe bestehend aus Schmerzmitteln, antiinfektiven Mitteln, Analgetika, entzündungshemmenden Mitteln, immunosuppressiven Mitteln, Steroiden, einschließlich Corticosteroiden, Glycoproteinen, Lipoproteinen und Kombinationen davon.

6. Knochenfüllerzusammensetzung gemäß Anspruch 5, wobei das Schmerzmittel ausgewählt ist aus der Gruppe bestehend aus Morphin, nichtsteroiden entzündungshemmenden Arzneimitteln, Oxycodon, Morphin, Fentanyl, Hydrocodon, Naproxyphen, Codein, Acetaminophen mit Codein, Acetaminophen, Benzocain, Lidocain, Procain, Bupivacain, Ropivacain, Mepivacain, Chloroprocain, Tetracain, Cocain, Etidocain, Prilocain, Procain, Clonidin, Xylazin, Medetomidin, Dexmedetomidin, VR1-Antagonisten und Kombinationen davon.

7. Knochenfüllerzusammensetzung gemäß Anspruch 6, wobei das Schmerzmittel Bupivacain ist.

8. Knochenfüllerzusammensetzung gemäß Anspruch 1, wobei der Knochenfüller zusätzlich eine osteoinduktive Komponente umfasst.

9. Knochenfüllerzusammensetzung gemäß Anspruch 8, wobei die osteoinduktive Komponente ausgewählt ist aus der Gruppe bestehend aus Zellbefestigungsmediatoren, peptidhaltigen Varianten der RGD-Integrinbindungssequenz, von der bekannt ist, dass sie die Zellanhaftung beeinflusst, biologisch aktiven Liganden, Integrinbindungssequenz, Liganden, knochenmorphogenen Proteinen, dem epidermalen Wachstumsfaktor, IGF-I, IGF-II, TGF-β I-III, Wachstumsdifferenzierungsfaktor, Parathyroidhormon, dem vaskulären endothelialen Wachstumsfaktor, Glycoprotein, Lipoprotein, bFGF, TGF-β-Überfamilie-Faktoren, BMP-2, BMP-4, BMP-6, BMP-12, BMP-14, Sonic Hedgehog, GDF6, GDF8, PDGF, Tenascin-C, Fibronectin, Thromboelastin, Thrombinabgeleiteten Peptiden und heparinbindenden Domänen.

10. Knochenfüllerzusammensetzung gemäß Ansprüchen 1, 2 oder 8, wobei das bioabbaubare Material 15 bis 75 Gewichtsprozent der Knochenfüllerzusammensetzung bildet.

## Revendications

1. Composition de comblement de cavité osseuse pour utilisation dans un procédé pour combler au moins une partie d'un volume de cavité osseuse dans un alésage osseux contenant un ancrage de suture, ledit ancrage ayant un corps d'ancrage, ledit corps d'ancrage ayant un volume, et ladite composition de comblement de cavité comprend un matériau biodégradable, **caractérisé en ce que** ledit procédé comprend la distribution de ladite composition dans ledit volume de cavité osseuse sous la forme d'un solide, ledit matériau biodégradable comprend un membre choisi dans le groupe constitué de l'hydroxyéthylcellulose et l'acide hyaluronique, et ladite composition comprend une quantité efficace d'un agent thérapeutique.

2. Composition de comblement de cavité osseuse selon la revendication 1, dans laquelle le comblement de cavité osseuse comprend en outre un composant ostéoconducteur

3. Composition de comblement de cavité osseuse selon la revendication 2, dans laquelle ledit composant ostéoconducteur est choisi dans le groupe constitué des phosphate tricalcique, phosphate alpha-tricalcique, phosphate bêta-tricalcique, carbonate de calcium, carbonate de baryum, sulfate de calcium, sulfate de baryum, hydroxyapatite, un polymorphe de phosphate de calcium, et des combinaisons de ceux-ci.

4. Composition de comblement de cavité osseuse selon la revendication 3 dans laquelle ledit composant ostéoconducteur est le phosphate bêta-tricalcique.

5. Composition de comblement de cavité osseuse selon la revendication 1 dans laquelle ledit agent thérapeutique est choisi dans le groupe constitué de médicaments antidouleurs, anti-infectieux, analgésiques, agents anti-inflammatoires, immunosupresseurs, stéroïdes, comprenant des corticostéroïdes, des glycoprotéines, des lipoprotéines, et des combinaisons de ceux-ci.

6. Composition de comblement de cavité osseuse selon la revendication 5 dans laquelle ledit médicament antidouleur est choisi dans le groupe constitué des morphine, médicaments anti-inflammatoires non stéroïdiens, oxycodone, morphine, fentanyle, hydrocodone, naproxyphène, codéine, acétaminophène avec codéine, acétaminophène, benzocaïne, lidocaïne, procaïne, bupivacaïne, ropivacaïne, mépivacaïne, chloroprocaïne, tétracaïne, cocaïne, étidocaïne, prilocaine, procaïne, clonidine, xylazine, médétomidine, dexmédétomidine, antagonistes de VR1, et des combinaisons de ceux-ci.

7. Composition de comblement de cavité osseuse selon la revendication 6 dans laquelle ledit médicament antidouleur est la bupivacaïne.

8. Composition de comblement de cavité osseuse selon la revendication 1, dans laquelle le comblement de cavité osseuse comprend en outre un composant ostéo-inducteur.

9. Composition de comblement de cavité osseuse selon la revendication 8, dans laquelle ledit composant ostéo-inducteur est choisi dans le groupe constitué de médiateurs de liaison cellulaire, variations contenant un peptide de la séquence de liaison d'intégrine RGD connu pour affecter la liaison cellulaire, ligands biologiquement actifs, séquence de liaison d'intégrine, ligands, protéines morphogénétiques osseuses, facteur de croissance épidermique, IGF-I, IGF-II, TGF-β I-III, facteur de différenciation de croissance, hormone parathyroïdienne, facteur de croissance de l'endothélium vasculaire, glycoprotéine, lipoprotéine, bFGF, facteurs de la superfamille TGF-β, BMP-2, BMP-4, BMP-6, BMP-12, BMP-14, Sonic hedgehog, GDF6, GDF8, PDGF, ténascine-C, fibronectine, thromboélastine, peptides dérivés de la thrombine, et domaines de liaison d'héparine.

10. Composition de comblement de cavité osseuse selon les revendications 1, 2 ou 8, dans laquelle ledit matériau biodégradable comprend de 15 à 75 pour cent en poids de la composition de comblement de cavité osseuse.
